# EUROPEAN PATENT APPLICATION

(11) **EP 1 498 736 A1**
(43) Date of publication of application: **19.01.2005**
(21) Application number: 04254308.2
(22) Date of filing: 16.07.2004
(51) Int. Cl.: G01N 35/10

(54) **Method for mixing a liquid in an analyzer**

(30) Priority: 18.07.2003 US 622258
(71) Applicant: Ortho-Clinical Diagnostics, Inc., Rochester, New York 14626 (US)
(72) Inventor: Jacobs, Merrit, Fairport, NY 14450 (US); Graham, Ed, Penfield, NY 14526 (US)
(74) Representative: Mercer, Christopher Paul

(57) **Abstract**

A method for mixing a liquid in a container in a diagnostic analyzer that includes: (a) providing a probe having a probe tip for aspirating and dispensing the liquid in the container; (b) providing the container containing one or more liquid(s) to be mixed; (c) inserting the probe into a first location of the container; (d) aspirating the one or more liquid(s) into the probe; (e) repositioning the probe or container to place the probe at a second location in the container; and (f) dispensing the one or more liquid(s) with the probe. A method of determining the amount of an analyte in a sample, includes the steps of: providing a sample containing an analyte in a container; providing a first reagent in the container; mixing the first reagent and sample as described above; optionally incubating the combined sample and reagent; optionally adding a second reagent to the container and mixing the second reagent and sample and first reagent as described above; and analyzing the sample for an analyte.

## Description

### BACKGROUND OF THE INVENTION

### Field of the Invention

The present invention relates to a process for improved mixing of a fluid in a diagnostic analyzer. In particular, the present invention relates to improved mixing that uses a aspirating/dispensing probe to conduct the mixing in multiple locations in a container.

### Description of the Related Art

Methods and systems for mixing liquids in containers that hold, e.g., samples being analyzed and reagents, such as analyzers for conducting clinical assays are known. For example, U.S. Patent No. 6,096,561 and U.S. Application No. 09/482,599 filed January 13, 2000 entitled "Failure Detection in Automatic Clinical Analyzers" describe diagnostic analyzers. Such systems typically contain a fluid aspirating and dispense probe that can include a disposable tip. The use of an aspirate/dispense probe to mix a liquid is known in the art. For example, U.S. Patent No. 5,773,305 and published U.S. Patent Application Nos. 2002/0081747 and 2002/0076826 describe such aspirate/dispense mixing. All of these documents are incorporated by reference in their entireties.

In mixing processes known in the art, the mixing is done with the tip of the probe at a fixed location in the container. This can lead to problems of incomplete mixing, particularly when the container has corners and does not have a parabolic shape to minimize corners. Such containers are described, for example, in U.S. Published application No. 2003/0003591 also incorporated by reference in its entirety. The result of incomplete mixing is a loss of precision and other deleterious results.

Washing slides used in diagnostic analyzers at different locations is also known in the art. See, e.g., U.S. Patent No. 5,641,688 and 5,620,860 also incorporated herein by reference in their entireties.

### SUMMARY OF THE INVENTION

One object of the invention is to overcome the disadvantages of the known art described above. Another object of the invention is to provide an improved mixing process for a liquid in a diagnostic analyzer. Still another object of the invention is to provide a process for mixing a liquid, such as sample and reagents, in a diagnostic analyzer to improve precision and other characteristics for the analyte being measured.

The foregoing and further objects of the invention are accomplished according to one aspect of the invention that provides a method for mixing a liquid in a container in a diagnostic analyzer that includes: (a) providing a probe having a probe tip for aspirating and dispensing the liquid in the container; (b) providing the container containing one or more liquid(s) to be mixed; (c) inserting the probe into a first location of the container; (d) aspirating the one or more liquid(s) into the probe; (e) repositioning the probe or container to place the probe at a second location in the container; and (f) dispensing the one or more liquid(s) with the probe.

According to another aspect of the invention there has been provided a method of determining the amount of an analyte in a sample, that includes the steps of: providing a sample containing an analyte in a container; providing a first reagent in the container; mixing the first reagent and sample as described above; optionally incubating the combined sample and reagent; optionally adding a second reagent to the container and mixing the second reagent and sample and first reagent as described above; and analyzing the sample for an analyte.

Further objects, features and advantages of the present invention will be apparent to those skilled in the art from detailed consideration of the preferred embodiments that follow.

### BRIEF DESCRIPTION OF THE DRAWINGS

Figure 1 is a schematic diagram showing the aspirate and dispense mixing sequence of a probe moving relative to a container according to a preferred embodiment.
Figure 2a is a schematic diagram showing a probe tip having a paddle shaped end.
Figure 2b is a top view of the probe tip shown in Figure 2a.

### DETAILED DESCRIPTION OF PREFERRED EMBODIMENTS

The present invention provides a method for mixing a liquid in a container used in a diagnostic analyzer, such as automated immunochemical analyzers. The method is applicable to any process in a diagnostic analysis that requires mixing of liquids, preferably two or more liquids, such as the mixing of sample and reagents, mixing of sample with diluent, mixing of multiple reagents, etc.

As noted above, the container that the liquids are mixed in can be anything capable of satisfactorily containing the liquids. The container can include materials such as plastic, glass, metal, etc. and can be configured as a cup, well, cuvette, test tube, etc. Those containers that will benefit most from the present invention are containers having corners, such as square-bottomed containers like those described in U.S. Publication No. 2003/0003591. That is, the present invention can be used in those shaped containers having various regions needing improved homogeneity. However, in some instances, even those containers having parabolic shape bottoms, such those cup-shaped containers described in U.S. Patent No. 5,441,895 (incorporated by reference in its entirety) will find benefit from the present invention.

In the present invention, the mixing is carried out by use of an aspirate/dispense probe. The probe may or may not be used to initially deposit the liquid in the container. As noted above, the use of the probe to mix liquids by aspirating and dispensing is known in the art and does not need further description. However, unlike known mixing techniques, in the present invention, the probe and container do not remain stationary relative to one another during the mixing process. That is, either the probe is repositioned (i.e., moves) at least once during the mixing process to a different location in the container, and/or the container is repositioned (i.e., moves) at least once during the mixing process, such that the probe is at a different location on the container. A combination of the probe and container moving is also envisioned.

The location that the probe moves or is repositioned to relative to the container can be either horizontal or vertical or a combination of both. Vertical movement or repositioning would be most useful in those embodiments where the container has a parabolic bottom.

There is at least one repositioning of the probe relative to the container during the mixing process, resulting in the probe being at two different positions in the container. However, as many movements as are necessary may be employed. Preferably, there may be two, three, four or five movements of the probe relative to the container resulting in the probe being at three, four, five and six locations in the container, respectively. Also, at each location, the probe may both aspirate and dispense to effect mixing. Alternatively, the probe may aspirate and dispense at different locations. For example, the probe may aspirate liquid at an initial position, then move to a second location before dispensing what it had aspirated at the first position. Combinations can also be employed. For example, the probe may both aspirate and dispense one or more times at a first location in the container. Then after a final aspirate, the probe containing aspirated liquid from the first location, is then moved to a second location where the probe dispenses the liquid it aspirated from the first position.

Additional mixing steps other than those described above may be used if they are necessary to enhance uniformity. The vertical height or locations of the tip during aspiration and dispense, along with the horizontal locations of each aspirate and dispense step may be optimized for the particular application and can be accomplished by those skilled in the art using the present specification as a guide. In a preferred embodiment, aspiration occurs near the top of the fluid to minimize wetted tip immersion and dispense occurs toward the bottom of the container with the tip lifting during the dispense to again minimize wetted tip depth.

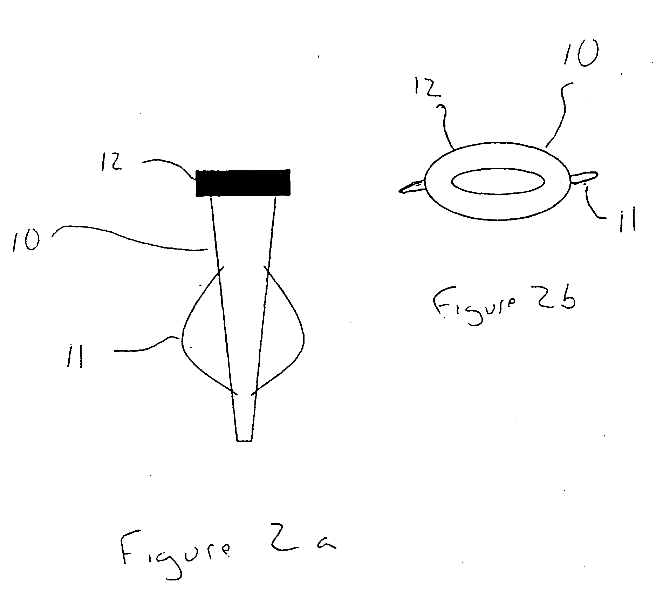

In another preferred embodiment, the dispense phase occurs at the corners of the cuvette to maximize the mixing in this area that is most difficult to mix. In another preferred embodiment, the tip can move from side to side during the dispense process or from back to front during the dispense process. This type of process has the additional advantage of using the tip as a paddle to create convective currents of the fluid in the container. Along these same lines, probe tips of specific geometry could be used to enhance the mixing process. Changes to the tips include a profile that fits into the cuvette more easily or a tip with surfaces that act as an improved paddle. Thus, in one single process the advantages of mixing using the pumping action of aspirate/dispense with a probe and the paddle-like mixing of the probe tip can be realized. Figure 2 shows a preferred embodiment where the probe tip is in the shape of a paddle to facilitate side to side mixing.

In those embodiments where a disposable tip is employed, the present mixing method enables a zero carryover (zero carryover because of the use of disposable tips) in containers that have sharp corners or other features that are likely to reduce mixing efficiency. When a disposable tip is employed, the tip is generally replaced between aspiration of each new liquid to prevent carryover. For example, a tip will be used to aspirate a sample and a new tip will be used to aspirate each reagent. However, in some instances, such as where the reagent supplies are single use, the same tip may be used. In those instances, it is preferred to replace the tips between each analysis.

The present method also provides a method of determining the amount of an analyte in a sample by using the mixing process of the present invention. The method includes providing the sample containing an analyte in a container, preferably a cuvette. The sample can be blood, plasma, etc. and can be added by the probe with a disposable tip. A new tip is preferably installed on the probe and a first reagent is dispensed to the cuvette. The mixing process according to the present invention is carried out, followed by incubating the sample and reagent, if necessary. If necessary, a second reagent is dispensed to the container, followed by mixing according to the present invention. The sample is then analyzed for the analyte. Methods for analyzing sample are known in the art and are described, for example, in EP-A-1 116 953. The analysis according to the present invention is particularly useful for measuring high density lipoprotein.

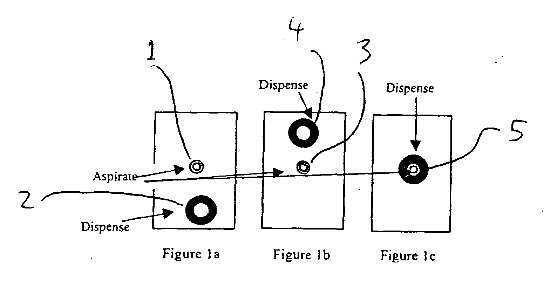

Now reference will be made with respect to the detailed description of the preferred mixing sequence shown in Figure 1a-c. In the embodiment shown in Figure 1a-c, the mixing is taking place in the same container, with the container being depicted at three different points in the mixing process. The container in Figure 1 is a cuvette having rectangular cross-section such as those described in U.S. Publication No. 2003/0003591.

Initially, the probe is brought into contact with the container at the position shown in reference numeral **1** as shown in Figure 1a. At position **1** (the center of the cuvette), the probe aspirates a selected quantity of the liquid into the probe tip. At this point, the probe moves to position **2** (front wall of the cuvette), where the liquid in the probe tip is then dispensed into the cuvette. The probe then moves to position **3** (center of the cuvette), as shown in Figure 1b, where fluid is again aspirated into the tip and then moves to position **4** (back wall of the cuvette) where the fluid aspirated at position **3** is dispensed. The probe then moves to position **5** (center of the cuvette), as shown in Figure 1c, where fluid is again aspirated into the tip. In this embodiment, the probe remains stationary at position **5** and dispenses the liquid. At this point, the liquid in the container is satisfactorily mixed and further aspirating and dispensing operations can be carried out on the aspirated liquid.

Figures 2a and 2b show a preferred embodiment where the probe tip is in the shape of a paddle to facilitate mixing. The probe **10** includes paddle portion **11** and top portion **12**. As shown in Figure 2b, the top of the probe **12** can be oval shaped to facilitate orienting the tip in the proper direction. The probe would likewise have to be configured in an oval shape if such an embodiment is employed. In another embodiment, the paddles could be configured in an X-shape to dispense with the need to orient the probe tip in any particular direction.

The mixing process according to the present invention can be implemented by a computer program, having computer readable program code, interfacing with the computer controller of the analyzer as is known in the art.

The present invention has proved to be particularly useful in improving the analysis performance of high density lipoprotein (HDL), in particular in improving the precision.

It will be apparent to those skilled in the art that various modifications and variations can be made to the compounds, compositions and processes of this invention. Thus, it is intended that the present invention cover such modifications and variations, provided they come within the scope of the appended claims and their equivalents.

The disclosure of all publications cited above are expressly incorporated herein by reference in their entireties to the same extent as if each were incorporated by reference individually.

## Claims

1. A method for mixing a liquid in a container in a diagnostic analyzer comprising:
(a) providing a probe having a probe tip for aspirating and dispensing the liquid in the container;
(b) providing the container containing one or more liquid(s) to be mixed;
(c) inserting the probe into a first location of the container;
(d) aspirating the one or more liquid(s) into the probe;
(e) repositioning the probe or container to place the probe at a second location in the container; and
(f) dispensing the one or more liquid(s) with the probe.

2. A method according to claim 1, further comprising:
(g) repositioning the probe or container to place the probe at a third location in the container;
(h) aspirating the one or more liquid(s) with the probe;
(i) repositioning the probe or container to place the probe at a fourth location in the container; and
(j) dispensing the one or more liquid(s) with the probe.

3. A method according to claim 2, further comprising:
(k) repositioning the probe or container to place the probe at a fifth location in the container;
(l) aspirating the one or more liquid(s) with the probe; and
(m) dispensing the one or more liquid(s) with the probe.

4. A method according to any one of claims 1 to 3, further comprising:
aspirating and dispensing the liquid at one location before repositioning the probe to another location.

5. A method according to any one of claims 1 to 4, wherein the repositioning is achieved by moving the probe.

6. A method according to any one of claims 1 to 4, wherein the repositioning is achieved by moving the container.

7. A method according to any one of claims 1 to 6, wherein the probe tip comprises a disposable tip, which is replaced before step (a).

8. A method according to any one of claims 1 to 7, wherein the container is a cuvette.

9. A method according to claim 8, wherein the cross-section of the cuvette is rectangular.

10. A method according to any one of claims 1 to 9, wherein the repositioning is horizontal.

11. A method according to any one of claims 1 to 9, wherein the repositioning is vertical.

12. A method according to any one of claims 1 to 11, wherein the probe tip is moved sideways to reposition the probe tip and the probe tip is disposable and has a flat side. In order to stir the fluid when the tip is moved sideways in the container.

13. A method according to claim 12, wherein the flat side is oriented to be perpendicular to the direction of movement of the probe tip.

14. A method according to any one of claims 1 to 13, implemented by a computer program interfacing with a computer.

15. A method of determining the amount of any analyte in a sample, comprising the steps of:
(a) providing a sample containing an analyte in a container;
(b) providing a first reagent in the container;
(c) mixing the first reagent and sample using the method of any one of claims 1 to 14;
(d) optionally incubating the combined sample and reagent;
(e) optionally adding a second reagent to the container and mixing the second reagent and sample and first reagent using the method of any one of claims 1 to 14; and
(f) analyzing the sample for an analyte.

16. A method according to clam 15, wherein the probe is used to dispense the sample, first and second reagent and a new probe tip is provided before each dispense of the sample, first and second reagent.

17. A method according to claim 15 or claim 16, wherein the analyte is high density lipoprotein.

18. An article of manufacture comprising a computer usable medium having computer readable program code configured to conduct the method of any one of claims 1 to 17.
